# EUROPEAN PATENT SPECIFICATION

(11) **EP 3 735 822 B1**
(45) Date of publication and mention of the grant of the patent: **15.09.2021**
(21) Application number: 19173537.2
(22) Date of filing: 09.05.2019
(51) Int. Cl.: A01G 7/04, C12M 1/00, C12N 1/12

(54) **METHOD FOR GROWING AQUATIC ORGANISMS CAPABLE OF PHOTOSYNTHESIS IN A CONTROLLED AQUEOUS ENVIRONMENT**
VERFAHREN ZUR ZÜCHTUNG VON ZUR PHOTOSYNTHESE FÄHIGEN AQUATISCHEN ORGANISMEN IN EINER KONTROLLIERTEN WÄSSRIGEN UMGEBUNG
PROCÉDÉ DE CROISSANCE D'ORGANISMES AQUATIQUES CAPABLES DE PHOTOSYNTHÈSE DANS UN ENVIRONNEMENT AQUEUX CONTRÔLÉ

(43) Date of publication of application: 11.11.2020
(73) Proprietor: P3 B.V., 7861 TB Oosterhesselen (NL)
(72) Inventor: STEL, Jarno, 7861 TB Oosterhesselen (NL); HULSHOFF, Hendrik Jan, 7861 TB Oosterhesselen (NL); VAN VELZEN, Dick, 7861 TB Oosterhesselen (NL); JAGER, Filips Gustaaf Hendrik, 7861 TB Oosterhesselen (NL)
(74) Representative: van Dam, Vincent

(56) References cited:
- EP-A1- 0 214 271
- CN-A- 108 192 801
- RU-C2- 2 644 261
- US-A1- 2016 014 974

## Description

The present invention relates to a method for growing aquatic organisms capable of photosynthesis in a controlled environment and a system for carrying out said method.

Organisms capable of photosynthesis convert light energy into chemical energy by converting carbon dioxide and water into the end-products oxygen and carbohydrates. This process is energized by light.

Algae form an example of such organisms, and are well-known as efficient producers of biomass. During photosynthesis, algae utilize carbon dioxide and light in the presence of water to produce oxygen and biomass. The algae produce lipids, vegetable oils and proteins which can be used for various purposes, for example as a source for human or animal nutrition, pharma or biofuel. Algae grow best under controlled conditions. For instance, algae are sensitive to temperature and light conditions, and in particular fluctuation thereof. Algal yield may be improved by controlling the growth parameters, such as temperature, CO₂ levels, light and nutrients.

The use of artificial light for plant growth is known, for instance by using light emitting diodes (LEDs).

The inventors have found that the present grow systems which make use of artificial light have their limitations with regard to efficiency, especially with regard to the high energy input.

US 2016/0014974 A1 discloses a stacked culture system for land plants and mentions exposing the plants to light cycles of 3 milliseconds of light followed by 3 milliseconds darkness.

EP 0 214 271 A1 discloses a plant for cultivating algae in liquid culture and suggests a relatively weak artificial illumination alternating with relatively long dark periods as favourable. EP 0 214 271 A1 discloses fluorescent lamps as light sources. The light flashes are disclosed to have a duration down to milliseconds. It is further disclosed in EP 0 214 271 A1 that the ratio of the time during which the algae in the liquid are kept in the dark to the time during which they are illuminated is 10:1.

CN 108192801A discloses a dynamic photobioreactor with a culture tank of light transmitting material, allowing natural light to pass through the culture tank and a method of cultivating microalgae using dynamic light, wherein the light quality, light intensity and light and dark frequency can be adjusted.

### Summary of the invention

The aim of the invention is to provide a method and system for growing organisms capable of photosynthesis under controlled conditions and which uses low energy input.

In one aspect the invention relates to a method for growing aquatic organisms capable of photosynthesis in accordance with claim 1.

In a second aspect the invention relates to a system for growing aquatic organisms capable of photosynthesis, in accordance with claim 5.

The method according to the first aspect can be carried out with the system according to the second aspect.

### Detailed description of the invention

The present invention is based on the finding that growing organisms capable of photosynthesis under conditions wherein the only source of light comes from light pulses which are controlled such that periods of light are interrupted by periods of complete darkness which highly reduces the amount of energy needed for growth of said organisms.

Photosynthesis is based on the capture of light photons by organisms capable of photosynthesis in order to convert light energy into chemical energy that can be used to generate biomass.

In nature sunlight is the light source for photosynthesis. Only a minor fraction of sunlight is converted to chemical energy and biomass. In fact, the inventors have observed that photosynthesizing organisms exposed to sunlight only utilize a very limited amount of this light for their activities. Estimations are that less than 13 % of the sunlight to which an organism is exposed can be converted to chemical energy and biomass. One reason for this lies in the fact that only light with specific wavelength can be utilized by the photosynthesis machinery. Another reason is that the kinetics of the photosynthesis mechanism require a certain lag time or down time between consecutive photons captured by an individual photosynthesis system. In order to explain this the kinetics of the photosynthesis mechanism will be discussed in summary in the following paragraphs.

Photons emitted from a light source are absorbed by antenna-like structures which are light-harvesting pigment complexes. This absorption only takes femto- to picoseconds. Next, excited energy states (so-called excitons) are transferred through so-called inter-protein hopping and magnetic resonance to the reaction centres of Photosystem II (PSII or reaction centre II or P680), causing the excitation of an electron. This takes 300-500 ps. It takes two electrons to in order to get PSII in reduced, i.e. 'closed' state, commonly referred to as P680*. Once PSII is in the 'closed' state, further excess photon energy cannot be transferred to the PSII. This excess photon energy is released through energy dissipation mechanisms and is as such lost for photosynthesis. This excess photon energy therefore will not be converted to chemical energy and biomass. Further now to the kinetics of the photosynthesis mechanism, the low redox state of P680* reduces primary electron acceptor pheophytin within 3-8 ps, so that PSII becomes oxidized (a state commonly referred to as P680+). Pheophytin passes the electron to plastoquinol, starting a flow of electrons down an linear electron transport chain including oxidation of plastoquinol by cytochrome b6f and leading eventually to the reduction of NADP to NADPH. This on its turn creates a proton gradient across the chloroplast membrane, which can be used by ATP synthase in the synthesis of ATP. ATP is used as energy source to drive many processes in living organisms. The PSII regains the electron it lost in the beginning of the process when a water molecule is split during process called photolysis. When PSII regains the electron it returns to the "opened state" and a subsequent photon can be captured again to generate an exciton. The slowest (3-5 ms) and therefore limiting step in the linear electron transfer chain is the oxidation of plastoquinol by cytochrome b6f, while the other processes take place in the order of picoseconds.

In other words, while it takes only light flashes with a duration in the femto/picosecond scale to produce excitons and thus trigger the photosynthesis cascade (referred herein as "excitation time"), it takes in the order of milliseconds (approximately 3-5 ms) before PSII reopens again so that the subsequent exciton capture may take place again (referred herein as "lag time"). During this millisecond lag time excess photon energy which cannot be used to produce excitons is released through energy dissipation mechanisms, such as photo reflection and heat, and is as such lost for photosynthesis. This excess photon energy therefore will not be converted to chemical energy and biomass. At the same time, excess exposure of PSII to light may lead to so-called photoinhibition. Photoinhibition comprises a series of reactions that inhibit various activities of PSII, leading to a decrease in photosynthesis efficiency. Photoinhibition is caused by damage to PSII due to light exposure. Photoinhibition occurs at all wavelengths. The more PSII is exposed to light, the more damage will occur and, consequently, the more photosynthesis efficiency will be adversely affected.

The provision of the method and system of the invention make it possible to use much less photon energy whilst still making optimal use of the photosynthesis machinery, because it allows to excite the photosynthesis machinery with a short light pulse while avoiding unnecessary and unusable photon energy by the provision of dark periods between pulses. Given the difference in duration between excitation time (fs/ps range) and lag time (ms) this results in a potential reduction of required light energy in multiple orders of magnitude.

It is essential that the course of growth of the organisms comprises dark periods between periods of light pulses in order to ensure periods of complete darkness in which all pigments will return to their resting state. In other words, at the end of a dark period the pigments of the photosynthesis machinery of organisms will all be reset to the opened state so that a short pulse of light will be able to activate a maximal amount of the available PSII molecules, thereby triggering a new chain of electron transport which leads to the reduction of NADP to NADPH, which on its turn enables the synthesis of ATP. If there were no dark periods, there would not be such a reset of the PSII molecules, leading to loss of photon energy via processes such as photoreflection and/or heat. Moreover, the above mentioned photoinhibition would take place at any moment of no complete darkness.

As mentioned above the lag time of the photosynthesis machinery and in particular PSII is approximately 3 to 5 ms. It is therefore preferred that the dark periods have a duration of at least 3 ms, more preferably at least 5 ms, in order to allow reset of all PSII molecules to avoid use of excess photon energy and to prevent the detrimental effects thereof. Therefore, the dark periods have a length in the order of milliseconds. More preferred, the dark periods have a length of at least 3 milliseconds. Most preferred the dark periods have a length of at least 5 milliseconds.

On the other hand, the excitation time of the photosynthesis machinery, or in other words the time it takes to capture a photon, is very short, namely in the order of femto/picoseconds. Therefore the length of the light pulses should be chosen as short as technically possible. Light sources that are well equipped for emitting very short pulses of light are diode based light sources. Light source drivers configured to control said diode based light sources to emit light pulses such that in said bioreactor periods of light are interrupted by periods of complete darkness, can be designed in accordance with methods known in the art.

The light sources are light emitting diodes (LEDs). LEDs are easy in use, can be designed to emit light of a specific wavelength and are relatively cheap. LED drivers that are capable of effecting light pulses in the picosecond to nanosecond range are available in the art. In the present invention, the dark periods are longer than the period of exposure to light. Although light pulses in the order of microseconds (1 µs - 999 µs) are possible to achieve the technical effects of the present invention and accordingly will already result in a marked decrease of required energy for photosynthesis, in the present invention the length of the light pulses is in the order of nanoseconds (1 ns - 999 ns) or less, for instance in the order of nanoseconds to picoseconds (1 - 999 ps). For instance the light pulses have a length of between 10 and 100 picoseconds.

Photosynthesizing organisms are only capable of using specific wavelength fractions from the natural light spectrum for photosynthesis. The spectrum used by organisms capable of photosynthesis is conventionally referred to as the Photosynthetically Active Radiation (PAR). The PAR region comprises wavelengths between 400-700 nm, which corresponds closely to the visible spectrum. Photosynthesis only occurs within the 400-500 and 600-700 nm range in the PAR region, so a large amount of light energy falls outside the PAR region and thus remains unusable. The exact optimal wavelengths differ per species of organism. For instance for most microalgae the major wavelengths usable for photosynthesis are within the range of 420-470 nm (blue) and/or 660-680 nm (red). The exact preference of wavelength and pulse length varies per species. In particular LEDs can be easily designed to emit a specific wavelength in accordance with methods known in the art. It is therefore preferred that said plurality of light sources comprises different types of light sources, each configured to emit light at a predetermined wavelength and pulse length, in particular with the values described above. In this case the light source driver is configured to control separately each type of light source to emit light pulses of a predetermined wavelength and pulse length. It is therefore possible to use multiple light sources for emitting light of multiple pulse lengths, as long as there are periods of complete darkness between consecutive pulses. Such a period of darkness may for instance be between the end of a pulse of a particular wavelength and the beginning of a pulse of another wavelength. A pulse of a particular length for a certain wavelength may for instance fall (time wise) within a pulse of a longer length for another wavelength. In the latter case the period of complete darkness will then be between consecutive pulses of the longer length. It can also be that first LEDs emitting a specific wavelength are configured to emit pulses in particular intervals, while further LEDs emitting another specific wavelength skip one or more of said intervals and are configured to emit a pulse coinciding with pulses of the first LEDs, but in a lower frequency.

The invention is applied to aquatic organisms. In this case the organisms are grown in an aqueous medium. In this case said organisms are preferably algae, such as micro-algae. In case of aquatic organisms the controlled environment is preferably an bioreactor. When in operation the bioreactor is filled with an aqueous medium containing organisms, for instance algae. In that case the system of the invention is a bioreactor for growing organisms capable of photosynthesis in an aqueous medium, wherein within said bioreactor a plurality of light sources is arranged and wherein the inside of said bioreactor is configured such that entry of light from a source outside said bioreactor can be completely prevented; and a light source driver, configured to control said light sources to emit light pulses, such that in said bioreactor periods of light are interrupted by periods of complete darkness. In a preferred embodiment such a bioreactor comprises multiple light bars comprising a plurality of distributed LEDs, the bars extending in the inside of the bioreactor. This way as much algae as possible will be exposed to the emitted light, which is advantageous for photosynthesis and thus the production of biomass.

## Claims

1. A method for growing aquatic organisms capable of photosynthesis within a controlled aqueous environment, wherein within said controlled environment a plurality of light sources is arranged, and wherein the inside of said controlled environment is configured such that light from a source outside said controlled environment does not enter said controlled environment;
wherein the method comprises controlling said light sources to emit light pulses,
exposing the organisms in said controlled environment to said light pulses, and
wherein periods of light are interrupted by periods of complete darkness;
**characterized in that**:
said light sources are light emitting diodes (LEDs);
**in that** the dark periods have a length in the order of milliseconds; and
**in that** the length of the light pulses is from 999 nanoseconds to 1 picosecond.

2. The method according to claim 1, wherein the dark periods have a length of at least 3 milliseconds, preferably at least 5 milliseconds.

3. The method for growing organisms according to the previous claims, wherein said plurality of light emitting diodes comprises different types of light emitting diodes, each configured to emit light at a predetermined wavelength and pulse length.

4. The method according to any of the previous claims, wherein said controlled environment is a bioreactor, and wherein said organisms algae, such as micro-algae.

5. A system for growing aquatic organisms capable of photosynthesis, comprising
a controlled aqueous environment, wherein within said controlled environment a plurality of light sources is arranged, and wherein the inside of said controlled environment is configured such that entry of light from a source outside said controlled environment can be completely prevented;
and a light source driver, configured to control said light sources to emit light pulses, such that in said controlled environment periods of light are interrupted by periods of complete darkness;
**characterized in that** the light source driver is further configured to control said light sources to emit light pulses such that the dark periods have a length in the order of milliseconds and the length of the light pulses is from 999 nanoseconds to 1 picosecond;
wherein said light sources are light emitting diodes (LEDs).

6. The system according to claim 5, wherein said plurality of light emitting diodes comprises different types of light emitting diodes, each configured to emit light at a predetermined wavelength and pulse length and wherein said light source driver is configured to control separately each type of light emitting diodes to emit light pulses of a predetermined wavelength and pulse length.

7. The system according to any of the claims 5 or 6, wherein said controlled environment is a bioreactor, and wherein said organisms are algae, such as micro-algae.

8. The system according to claim 7, wherein the bioreactor comprises multiple light bars comprising a plurality of distributed LEDs, the bars extending in the inside of the bioreactor.

## Patentansprüche

1. Verfahren zum Züchten von Wasserorganismen, die zur Fotosynthese fähig sind, innerhalb einer kontrollierten wässrigen Umgebung, wobei innerhalb der kontrollierten Umgebung eine Mehrzahl von Lichtquellen angeordnet ist, und wobei das Innere der kontrollierten Umgebung so gestaltet ist, dass Licht von einer Quelle außerhalb der kontrollierten Umgebung nicht in die kontrollierte Umgebung eintritt;
wobei das Verfahren das Steuern der Lichtquellen zum emittieren von Lichtpulsen umfasst,
das Aussetzen der Organismen in der kontrollierten Umgebung gegenüber den Lichtpulsen, und
wobei Perioden von Licht durch Perioden von vollständiger Dunkelheit unterbrochen werden,
**dadurch gekennzeichnet,**
**dass** die Lichtquellen Licht emittierende Dioden (LEDs) sind,
**dass** die Dunkelperioden eine Länge in der Größenordnung von Millisekunden haben, und
**dass** die Länge der Lichtpulse im Bereich von 999 Nanosekunden bis 1 Picosekunde beträgt.

2. Verfahren nach Anspruch 1, wobei die Dunkelperioden eine Länge von mindestens 3 Millisekunden, bevorzugt mindestens 5 Millisekunden haben.

3. Verfahren zum Züchten von Organismen nach den vorhergehenden Ansprüchen, wobei die Mehrzahl der lichtemittierenden Dioden lichtemittierenden Dioden verschiedenen Typs umfasst, die jeweils so eingerichtet sind, dass sie Licht mit einer vorbestimmten Wellenlänge und Pulslänge emittieren.

4. Verfahren nach einem der vorhergehenden Ansprüche, wobei die kontrollierte Umgebung ein Bioreaktor ist, und wobei die Organismen Algen, beispielsweise Mikroalgen sind.

5. System zum Züchten von Wasserorganismen, die zur Fotosynthese fähig sind, umfassend eine kontrollierte wässrige Umgebung, wobei innerhalb der kontrollierten Umgebung eine Vielzahl von Lichtquellen angeordnet ist, und wobei das Innere der kontrollierten Umgebung so eingerichtet ist, dass der Eintritt von Licht von einer Quelle außerhalb der kontrollierten Umgebung vollständig verhindert werden kann;
und einen Lichtquellentreiber, der so eingerichtet ist, dass er die Lichtquellen zum Emittieren von Lichtpulsen steuert, derart,
dass in der kontrollierten Umgebung Perioden von Licht durch Perioden von vollständiger Dunkelheit unterbrochen werden,
**dadurch gekennzeichnet, dass** der Lichtquellentreiber ferner so eingerichtet ist, dass er die Lichtquellen so steuert, dass sie Lichtpulse emittieren, sodass die Dunkelperioden eine Länge in der Größenordnung von Millisekunden haben und die Länge der Lichtpulse im Bereich zwischen 999 Nanosekunden bis 1 Picosekunde liegt.

6. System nach Anspruch 5, wobei die verschiedenen Typen von lichtemittierenden Dioden lichtemittierende Dioden verschiedenen Typs beinhalten, die jeweils so eingerichtet sind, dass sie Licht einer vorgegebenen Wellenlänge und Pulslängen emittieren, und wobei der Lichtwellentreiber so eingerichtet ist, dass er jeden Typ von lichtemittierenden Dioden separat steuert, um Lichtpulse einer vorbestimmten Wellenlänge und Pulslänge zu emittieren.

7. System nach einem der Ansprüche 5 oder 6, wobei die kontrollierte Umgebung ein Bioreaktor ist und wobei die Organismen Algen, insbesondere Mikroalgen sind.

8. System nach Anspruch 7, wobei der Bioreaktor eine Vielzahl von Lichtleisten umfasst, die eine Vielzahl von verteilten LEDs umfassen, wobei sich die Leisten im Inneren des Bioreaktors erstrecken.

## Revendications

1. Procédé de culture d'organismes aquatiques capables de photosynthèse dans un environnement aqueux contrôlé, dans lequel, dans ledit environnement contrôlé, une pluralité de sources de lumière est disposée, et dans lequel l'intérieur dudit environnement contrôlé est configuré de sorte que la lumière qui provient d'une source située à l'extérieur dudit environnement contrôlé ne pénètre pas dans ledit environnement contrôlé ;
dans lequel le procédé comprend le contrôle desdites sources de lumière afin d'émettre des impulsions de lumière,
l'exposition des organismes dans ledit environnement contrôlé auxdites impulsions de lumière, et
dans lequel les périodes de lumière sont interrompues par des périodes d'obscurité totale ;
**caractérisé en ce que** :
lesdites sources de lumière sont des diodes électroluminescentes (LED) ;
**en ce que**
les périodes d'obscurité possèdent une durée de l'ordre de quelques millisecondes ; et
**en ce que**
la longueur des impulsions de lumière est comprise entre 999 nanosecondes et 1 picoseconde.

2. Procédé selon la revendication 1, dans lequel les périodes d'obscurité possèdent une durée d'au moins 3 millisecondes, de préférence d'au moins 5 millisecondes.

3. Procédé de culture d'organismes selon les revendications précédentes, dans lequel ladite pluralité de diodes électroluminescentes comprend différents types de diodes électroluminescentes, chacune configurée pour émettre de la lumière à une longueur d'onde et une durée d'impulsion prédéterminées.

4. Procédé selon l'une quelconque des revendications précédentes, dans lequel ledit environnement contrôlé est un bioréacteur, et dans lequel lesdits organismes sont des algues, comme des microalgues.

5. Système de culture d'organismes aquatiques capables de photosynthèse, comprenant
un environnement aqueux contrôlé, dans lequel, dans ledit environnement contrôlé, une pluralité de sources de lumière est disposée, et dans lequel l'intérieur dudit environnement contrôlé est configuré de sorte que l'entrée de lumière qui provient d'une source située à l'extérieur dudit environnement contrôlé puisse être complètement empêchée ;
et un système de commande des sources de lumière, configuré pour contrôler lesdites sources de lumière afin d'émettre des impulsions de lumière, de sorte que, dans ledit environnement contrôlé, les périodes de lumière soient interrompues par des périodes d'obscurité totale ;
**caractérisé en ce que** le système de commande des sources de lumière est en outre configuré pour contrôler lesdites sources de lumière afin d'émettre des impulsions de lumière de sorte que les périodes d'obscurité possèdent une durée de l'ordre de quelques millisecondes et que la durée des impulsions de lumière soit comprise entre 999 nanosecondes et 1 picoseconde ;
dans lequel lesdites sources de lumière sont des diodes électroluminescentes (LED).

6. Système selon la revendication 5, dans lequel ladite pluralité de diodes électroluminescentes comprend différents types de diodes électroluminescentes, chacune configurée pour émettre de la lumière à une longueur d'onde et une durée d'impulsion prédéterminées, et dans lequel ledit système de commande des sources de lumière est configuré pour contrôler séparément chaque type de diodes électroluminescentes afin d'émettre des impulsions de lumière d'une longueur d'onde et d'une durée d'impulsion prédéterminées.

7. Système selon l'une quelconque des revendications 5 ou 6, dans lequel ledit environnement contrôlé est un bioréacteur, et dans lequel lesdits organismes sont des algues, comme des microalgues.

8. Système selon la revendication 7, dans lequel le bioréacteur comprend plusieurs barres de lumière comprenant une pluralité de LED distribuées, les barres s'étendant à l'intérieur du bioréacteur.
